# EUROPEAN PATENT APPLICATION

(11) **EP 1 759 662 A1**
(43) Date of publication of application: **07.03.2007**
(21) Application number: 06254322.8
(22) Date of filing: 17.08.2006
(51) Int. Cl.: A61F 2/18

(54) **Crimp assist middle ear prosthesis**

(30) Priority: 06.09.2005 US 219950
(71) Applicant: Clarity Corporation, Memphis, TN 38133 (US)
(72) Inventor: Prescott, Anthony D., Arlington 38002, Tenessee (US); Reitan, Harlan J., Collierville 38017, Tenessee (US)
(74) Representative: Johnson, Terence Leslie

(57) **Abstract**

A middle ear prosthesis (34) comprises a member adapted to supplement an ossicular bone when implanted in an ear. An anatomically conforming structure is secured to the member comprising an upper arcuate portion (42) and a lower arcuate portion (48), each having near (44, 50) and distal (46, 52) ends respectively. A crimp assist portion (54) is connected to the near ends of the upper and lower arcuate portions so that the upper and lower arcuate portions (42, 48) generally define portions of a circle for partially surrounding a bone. The crimp assist portion (54) extends outside of the circle. The crimp assist portion (54) is of a crimpable metal capable of retaining different shapes so that the crimp assist portion can be crimped to close the structure around the bone without crimping within the circle.

## Description

### FIELD OF THE INVENTION

This invention relates to an ossicular prosthesis used for replacement and reconstruction and, more particularly, to a crimp assist middle ear prosthesis.

### BACKGROUND OF THE INVENTION

Due to disease, trauma, or congenital malformation, the ossicles of the middle ear are sometimes damaged. Otosclerosis is a form of hearing loss caused by the immobilization of the stapes bone in the middle ear. A stapedotomy is a surgical procedure to correct for this condition and involves removing a portion of the stapes bone, drilling a tiny hole in the footplate of the stapes, through the oval window, and connecting a small piston-like prosthesis to another bone in the middle ear with a wire crimp. With such a prosthesis, sound vibrations can propagate through to the inner ear, restoring hearing.

There are currently two common types of stapes prosthesis used. One is referred to as a piston prosthesis and the other is referred to as a bucket handle prosthesis. The function of either prosthesis is to restore continuity between the incus and inner ear fluids. Traditional piston type stapes prostheses consist mainly of a small gauge wire or ribbon formed in the shape of a hook mated to a larger diameter shaft. These types of prostheses are placed over the long process of the incus, which is no longer attached to the stapes superstructure, and through the drilled hole in the stapes footplate. A hook portion of the wire is crimped to the incus bone by the surgeon using a forceps type instrument. In this procedure, the jaws of the forceps actually are pressed against the incus bone. This action of crimping the wire to the incus is subjective and may lead to a condition where the crimp is either too loose or too tight. If the crimp is too tight, then blood supply to the incus may be hindered and a condition known as necrosis occurs. In necrosis, the portion of the incus bone below the crimp eventually dies and erodes. Once the bone erodes, then the piston wire will fall from the bone and a revision surgery is required. If the crimp is too loose, then the prosthesis may not transfer sound bite vibrations efficiently because the coupling to the bone does not provide good contact. As is apparent, this crimping is a critical component of the stapedotomy surgery.

A common complication of existing stapes prosthesis is necrosis of the incus bone after the prosthesis has been crimped to the incus. The resulting effect is that the blood supply of the incus below the crimped prosthesis slowly diminishes until the bone erodes. Usually, a revision surgery is required and the underlying problem is that the incus bone is now shorter due to erosion. Depending on how much of the incus remnant remains, a stapes prosthesis may be used again but often the incus is too short for this type of prosthesis.

Fitting a prosthesis to an eroded short incus is problematic for at least two reasons. First, the surgeon never knows how much of the incus is missing. Second, the incus exhibits a tapered shape which is difficult to attach a prosthesis to the more superior position or towards where the incus joins the malleus. Any such prosthesis used for this indication requires crimping to the incus remnant.

Similar problems can occur with other middle ear prosthesis which rely on crimping. For example, a prosthesis referred to as a "partial" can be used to connect the stapes to the tympanic membrane.

The present invention is directed to improvements in crimpable type prostheses.

### SUMMARY OF THE INVENTION

In accordance with the invention, there is provided a crimp assist middle ear prosthesis.

Broadly, there is disclosed in accordance with one aspect of the invention, a middle ear prosthesis comprising a member adapted to supplement an ossicular bone when implanted in an ear. A n anatomically conforming structure is secured to the member comprising an upper arcuate portion and a lower arcuate portion, each having near and distal ends. A crimp assist portion is connected to the near ends of the upper and lower arcuate portions so that the upper and lower arcuate portions generally define portions of a circle for partially surrounding a bone of the middle ear. The crimp assist portion extends outside of the circle. The crimp assist portion is of a crimpable metal capable of retaining different shapes so that the crimp assist portion can be crimped to close the structure around the bone of the middle ear without crimping within the circle.

It is a feature of the invention that the structure comprises a titanium wire. More particularly, the crimp assist portion is made from a relatively soft unalloyed titanium.

It is another feature of the invention that the crimp assist portion is angled at about 45° relative to an axis of the shaft.

It is still another feature of the invention that the distal ends of the arcuate portions are spaced apart and are flared away from one another to define an enlarged opening.

It is still another feature of the invention that the crimp assist portion is generally U-shaped.

There is disclosed in accordance with another aspect of the invention a middle ear prosthesis comprising a member adapted to supplement an ossicular bone when implanted in an ear. An anatomically conforming structure is secured to the member comprising an upper arcuate portion and a lower arcuate portion, each having near and distal ends. A crimp assist portion is connected to the near ends of the upper and lower arcuate portions so that the upper and lower arcuate portions generally define portions of a circle for partially surrounding a bone of the middle ear. The crimp assist portion extends outside of the circle. The circle in a pre-implant state has a diameter greater than a diameter of a portion of the bone of the middle ear so that the crimp assist portion can be crimped to close the structure around the bone of the middle ear without crimping within the circle and after crimping the circle has a diameter to be retained on the bone of the middle ear.

There is disclosed in accordance with a further aspect of the invention the method of implanting a middle ear prosthesis comprising removing a portion of an ossicular chain of an ear; providing a middle ear prosthesis comprising a member adapted to replace an ossicular bone, and an anatomically conforming structure secured to the member comprising an upper arcuate portion and a lower arcuate portion, each having near and distal ends, and a crimp assist portion connected to the near ends of the upper and lower arcuate portions so that the upper and lower arcuate portions generally define portions of a circle for partially surrounding a remnant bone of the middle ear and the crimp assist portion extending outside of the circle, the structure being of a crimpable metal capable of retaining different shapes; and implanting the prosthesis in the ear by placing the structure over the remnant bone of the middle ear and crimping the crimp assist portion to close the structure around the remnant bone of the middle ear without crimping within the circle.

Further features and advantages of the invention will be readily apparent from the specification and from the drawings.

### BRIEF DESCRIPTION OF THE DRAWINGS

Fig. 1 is a perspective, partial cross-section view of the anatomy of an ear showing a normal ossicular chain;
Fig. 2 is a view, similar to that of Fig. 1, showing the middle ear with part of the ossicular chain removed and a drill being used to drill a hole through a footplate of the stapes and an oval window;
Fig. 3 is a view, similar to that of Fig. 1, showing the middle ear with part of the stapes removed and replaced with a prosthesis in accordance with the invention;
Fig. 4 is an elevation of a crimp assist piston prosthesis in accordance with the invention;
Fig. 5 is a perspective view illustrating positioning of the prosthesis of Fig. 4 in the middle ear;
Fig. 6 is an elevation view illustrating crimping the prosthesis in the middle ear;
Fig. 7 is a plan view of that which is illustrated in Fig. 6;
Fig. 8 is a perspective view, similar to Fig. 5, illustrating the prosthesis crimped around the incus after implantation;
Fig. 8A is a plan view illustrating an alternative prosthesis with an out of plane crimp assist portion crimped around the incus after implantation;
Fig. 9 is a perspective view of a middle ear prosthesis for crimping to both an incus and a head of a stapes;
Fig. 10 is a perspective view illustrating crimping the prosthesis of Fig. 9 in the middle ear;
Fig. 11 is a perspective view of a middle ear prosthesis for crimping to the head of a stapes and including a series of loops for crimping to an incus;
Fig. 12 is a perspective view illustrating crimping the prosthesis of Fig. 11 in the middle ear;
Fig. 13 is a perspective view of a middle ear prosthesis, similar to Fig. 11, using a crimpable cage;
Fig. 14 is a perspective view illustrating crimping the prosthesis of Fig. 13 in the middle ear;
Fig. 15 is a perspective view of an adjustable length partial middle ear prosthesis having a crimp assist loop in accordance with the invention;
Fig. 16 is a perspective view illustrating crimping the prosthesis of Fig. 15 in the middle ear;
Fig. 17 is a perspective view illustrating a middle ear prosthesis including a cup and a hook;
Fig. 18 is a bottom plan view of the prosthesis of Fig. 17;
Fig. 19 is a perspective view of a middle ear prosthesis including a cup and hook according to an alternative embodiment of the invention;
Fig. 20 is a side elevation view of the prosthesis of Fig. 19;
Fig. 21 is a perspective view illustrating a prosthesis crimped around the incus after implantation, the prosthesis including an offset shaft pivotally connected to a piston;
Fig. 22 is a perspective view illustrating a middle ear prosthesis including a can and two hooks in accordance with the invention;
Fig. 23 is an elevation view of the prosthesis of Fig. 22; and
Fig. 24 is a top plan view of the prosthesis of Fig. 22 with parts removed for clarity.

### DETAILED DESCRIPTION OF THE INVENTION

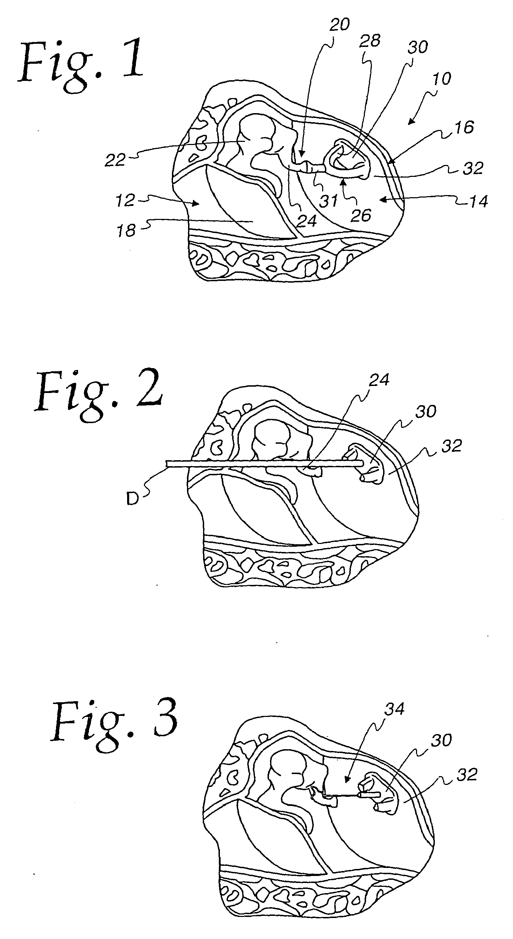

Referring initially to Fig. 1, a human ear 10 includes an external or outer ear 12, a middle ear 14 and an inner ear 16. A tympanic membrane 18, also called the ear drum, separates the outer ear 12 from the middle ear 14. The middle ear 14 includes an ossicular chain 20 comprising three small bones that are connected and transmit the sound waves from the ear drum 18 to the inner ear 16. The three small bones are called the malleus 22, the incus 24, and the stapes 26. The stapes 26 includes a loop or arch 28 connected to a footplate 30. The stapes 26 has a head 31 connected to the incus 24. The footplate is positioned on an oval window 32 that separates the middle ear 14 from the inner ear 16.

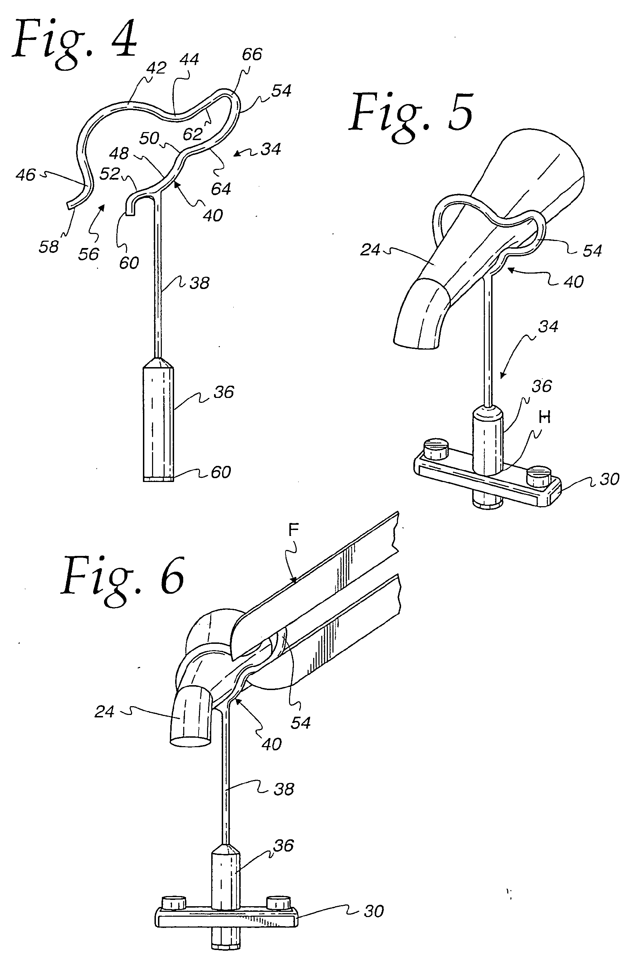

In accordance with one aspect of the invention, a crimp assist piston type middle ear prosthesis 34, see Fig. 4, is used for treating otosclerosis or the like, i.e. diseases that require removal of the stapes. The procedure for treating such conditions in accordance with the invention is for a surgeon to initially remove a portion of the ossicular chain between the footplate 30 and the incus 24, namely a part of the stapes 26, as shown in Fig. 2. A drill bit D, or a laser, not shown, may be used to create a hole through the footplate 30 and the oval window 32 to receive the prosthesis 34. Fig. 3 illustrates the prosthesis 34 in place after implantation.

Referring to Fig. 4, the prosthesis 34 includes a pedestal base or piston 36 and a shaft 38 extending from the piston 36. The shaft 38 is coaxial with the piston 36. The shaft 38 and the piston 36 are adapted to replace the stapes 26. An anatomically conforming structure 40, also referred to herein as a hook, is secured to the shaft 38 opposite the piston 36. Particularly, the hook 40 is laser welded to the shaft 38. The shaft 38 is molded to the base 36. The hook 40 comprises an upper arcuate portion 42 having a near end 44 and a distal end 46 and a lower arcuate portion 48 having a near end 50 and a distal end 52. A crimp assist portion 54 is connected between the upper arcuate portion near end 44 and the lower arcuate portion near end 50 so that the arcuate portions 42 and 48 generally define portions of a circle. Particularly, the arcuate portions 42 and 48 generally define arcs positioned relative to one another to partially surround a space in the form of a circle, oval or the like, as is apparent. For simplicity herein this shape is hereinafter described as a circle. The distal ends 46 and 52 are spaced from one another to provide an opening 56. Advantageously, the distal ends 46 and 52 are flared away from one another as at 58 and 60, respectively, to provide an enlarged opening 56.

The crimp assist portion 54 is angled at about 45° relative to an axis of the shaft 38 and piston 36 and is located outside of the circle discussed above. This locates the crimp assist portion 54 in a position to allow better access for a surgeon. The crimp assisted portion 54 includes opposite legs 62 and 64 that connect to the respective upper arcuate portion 42 and lower arcuate portion 48 and are generally equidistant from one another moving away from the arcuate portions 42 and 48 and then diverged towards one another where they join at a vertex 66. As is apparent, the hook 40, from the side, resembles the head of an animal, with the opening 56 resembling the mouth and the crimp assist portion 54 resembling an ear.

In accordance with the invention, the hook 40 may be of a one piece wire of a crimpable metal capable of retaining different shapes. Alternatively, the crimp assist portion 54 may be of a different material than the arcuate portions 42 and 48, with the different portions being welded together. In an exemplary embodiment of the invention, the prosthesis 34 is formed of an unalloyed titanium for surgical implant applications. For example, the hook 40 may be made of a titanium that satisfies the specifications of AFTM standard F67. This standard identifies four grades of unalloyed titanium for surgical implant applications. The grades are numbered 1, 2, 3 and 4. The grades range from 1 which is a relatively soft titanium that is readily malleable to grade 4 which is relatively hard and more spring-like. Advantageously, the hook 40, or at least the crimp assist portion 54, is made of grade 1 titanium to be malleable. Grades 3 and 4 provide too much resistance to deformation and act more spring-like and thus would not be satisfactory as a crimpable material. Grade 2 titanium could also be used, but is not as readily malleable as grade 1 titanium. In another example, the crimp assist portion 54 could be of grade 1 or 2 titanium and the arcuate portions 42 and 48 of grade 3 or 4 titanium. As is apparent, the prosthesis 34 may be of a composite design using different materials for the shaft 38 and/or the piston 36, as these elements are not crimped.

In an illustrative embodiment of the invention, the circle defined by the hook 40 has a diameter of about 1 mm. The wire used for the hook 40 and the shaft 38 is typically .005" to .007" in diameter. The hook 40 may also be in the form of a ribbon or flattened wire having a dimension of approximately .004" x .010". The piston 36 is typically .4 mm., .6mm, or .8 mm. in diameter.

The base 36 has a depth indicator 60, see Fig. 4, to assist in visualization of foot plate penetration. The indicator 60 may be about 0.5 mm. in length and may comprise an anodized section at the distal end of the base 36.

Fig. 5 illustrates the prosthesis 34 during an initial stage of implantation with the piston 36 inserted through a hole H in the footplate 30, and likewise the oval window 32, see Fig. 3, and the hook 40 placed over the long process of the incus 24. In this pre-implant state, the circle has a diameter greater than a diameter of remnant portion of the incus 24 to which it will be crimped, as is apparent.

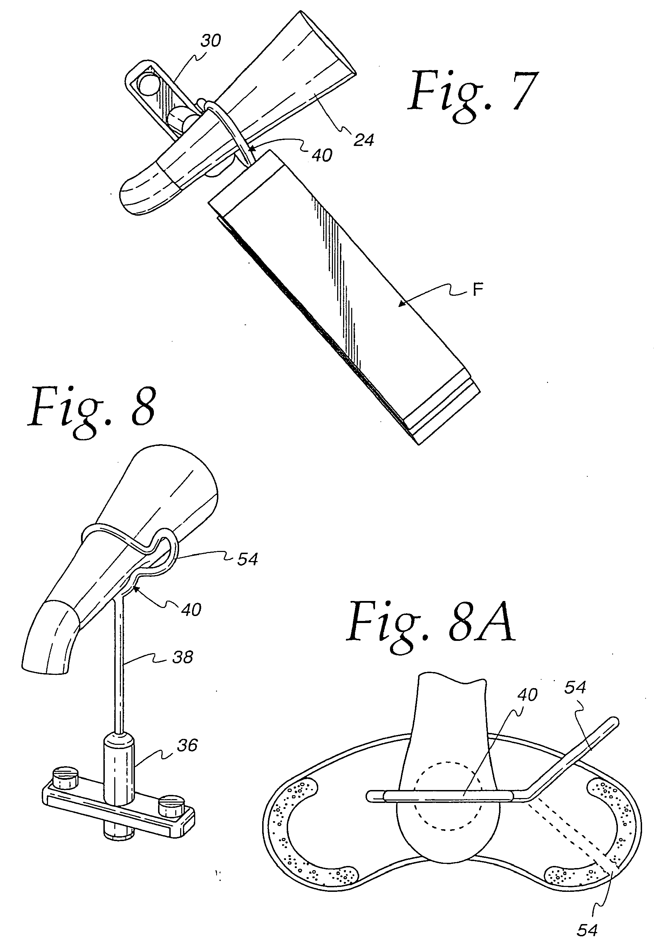

Referring to Figs. 6 and 7, a forceps F or the like is used to grasp the crimp assist portion 54 to crimp the hook 40 to close it around the incus 24. Because the crimp assist portion 54 extends outside of the circle defined by the arcuate portions 42 and 48, the forceps F provide crimping action outside of the circle so the forceps do not provide crimping pressure directly to the bone. Particularly, pressure from the forceps F is not applied directly over the bone but only to the crimp assist portion 54 which collapses the circle so that the arcuate portions 42 and 48 define a smaller circle having a diameter to be retained on the remnant incus, as shown in Fig. 8. Thereafter, the crimp assist portion 54 retains the crimped shape, see Fig. 8, due to the malleability of the titanium. Thus, by providing an extended crimp assist portion 54 to the hook 40, a means is provided for the surgeon to close the wire around the remnant incus bone without placing undesired forces upon the bone itself. Subjectivity of the crimp is addressed because the forces used by the surgeon to close the extended loop do not place undesired forces upon the remnant incus bone itself. Rather, the wire closes around the remnant incus bone from the extended portion being closed with the forceps, as is apparent in comparing the crimp assist portion 54 in Fig. 5 relative to Fig. 8.

Fig. 8A illustrates an alternative arrangement in which the crimp assist portion 54 is out of plane with the hook 40, in either of two directions, as shown in solid and dashed line.

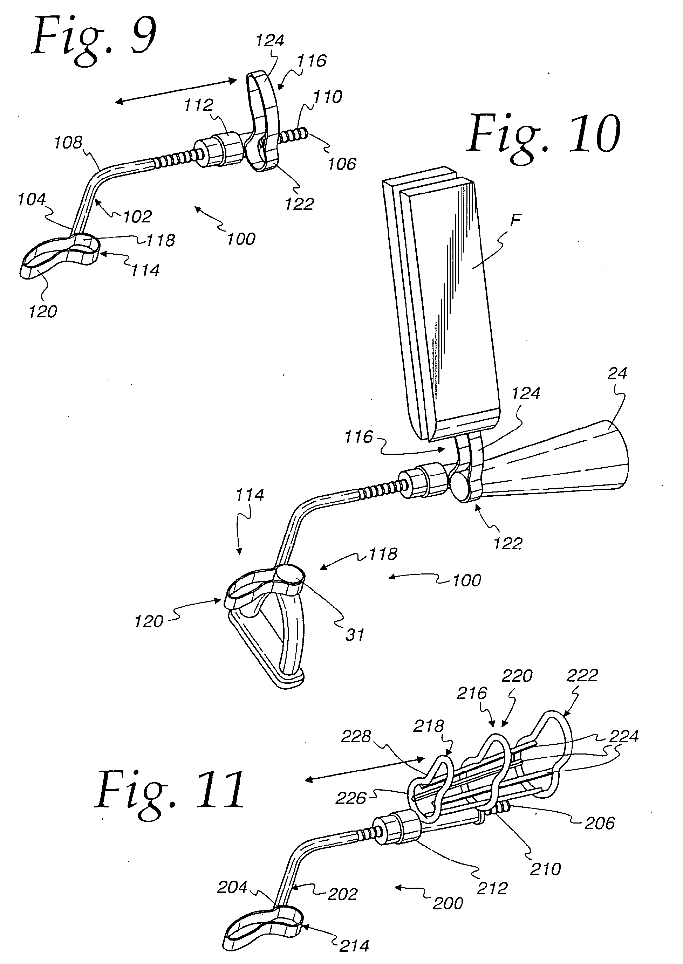

Referring to Fig. 9, a prosthesis 100 is adapted for use in necrosis applications, such as after a prior prosthesis has been crimped to the incus and it must be fit to an eroded short incus. The prosthesis 100 includes an elongate shaft 102 having a first end 104 and a second end 106. The shaft 102 is bent as at 108. As such, the shaft 102 is adapted to replace a portion of a head of the stapes and a portion of the incus. As is apparent, the shaft 102 can be formed of a malleable material so that it can be bent as necessary for implantation. The shaft 102 has a corrugated portion 110 between the bend 108 and the second end 106. A flexible sleeve 112 is received on the corrugated portion 110 so that it can be adjustably positioned. Particularly, the adjustability is generally similar to that described in Prescott U.S. Patent No. 6,168,625, the specification of which is incorporated by reference herein.

A hook in the form of a first loop 114 is secured to the shaft 102 at the first end 104. A hook in the form of second loop 116 is secured to the sleeve 112. Each loop 114 and 116 is generally similar in shape, although they may be of different sizes. Each of the loops 114 and 116 is generally similar to the hook 40 discussed above. Distal ends of arcuate portions can be joined together to define a closed loop, as shown, or alternatively can be open, as desired. In the illustrated embodiment, the first loop 114 includes a circular portion 118 and a crimp assist portion 120. Similarly, the second loop 116 includes a circular portion 122 and a crimp assist portion 124.

Referring to Fig. 10, implantation of the prosthesis 100 is illustrated. The first loop 114 is placed over the stapes head 31 which is received in the circular portion 118. The flexible sleeve 112 can be adjusted, as indicated by the arrow in Fig. 9, so that the second loop 116 is positioned so that its circular portion 122 is received on the remnant eroded short incus 24. A crimper or forceps F are then used to crimp the second loop crimp assist portion 124, as shown, and likewise the first loop crimp assist portion 120. As with the above described example, pressure from the forceps F is not applied directly over the bone but only to the crimp assist portions 116 and 124.

In the illustrated embodiment of the invention, the loops 114 and 116 are formed of a ribbon or flattened wire, as discussed above.

Referring to Fig. 11, a prosthesis 200 in accordance with another embodiment of the invention is illustrated also for incus necrosis applications. The prosthesis 200 includes a shaft 202 having opposite first ends 204 and second ends 206. The shaft 202 is generally similar to the shaft 102, discussed above, relative to Fig. 9. A first loop 208 is provided at the first end 204. The shaft 202 has a corrugated portion 210 at the second end 206. A flexible sleeve, such as a silicon sleeve, 212 is adjustably received on the corrugated portion 210, as with the embodiment of Fig. 9. In this embodiment, the sleeve 212 is elongated and is secured to a hook in the form of a cage 216. The cage 216 includes three loops 218, 220 and 222 of successively larger sizes. The loops 218, 220 and 222 are secured together using elongate bars 224. Each of the loops 218, 220 and 222 are formed of wire and are otherwise similar to the loop 116, discussed above. Particularly, as with the loop 116, each includes a circular portion 226 for surrounding a portion of a remnant incus and a crimp assist portion 228. For clarity only portions of the loop 218 are numbered in the drawing. The cage 216 can be adjusted to the tapered shape of a remnant eroded incus. Implantation is generally similar to that discussed above relative to Fig. 10, except that the forceps F are used to crimp the crimp assist portion 228 of each of the loops 218, 220 and 222 around the incus 24.

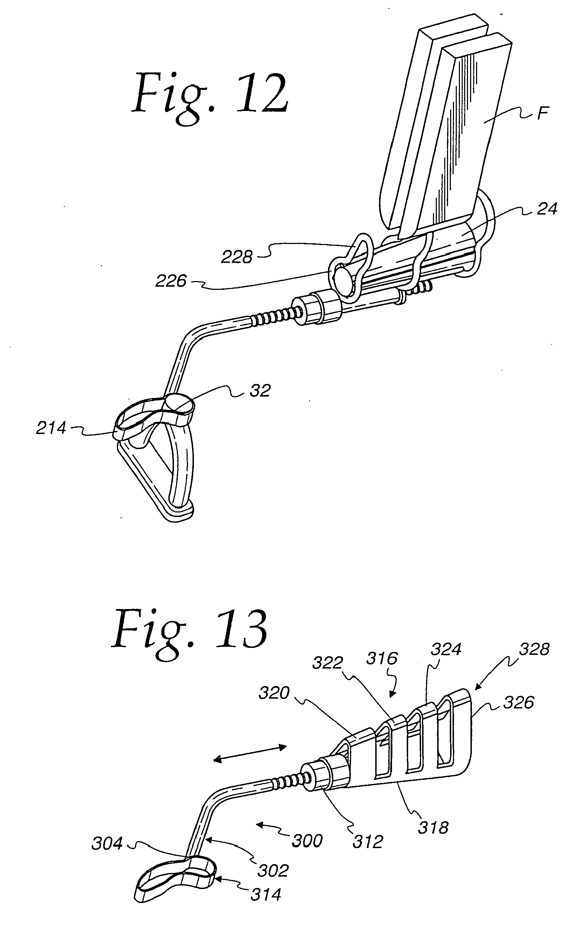

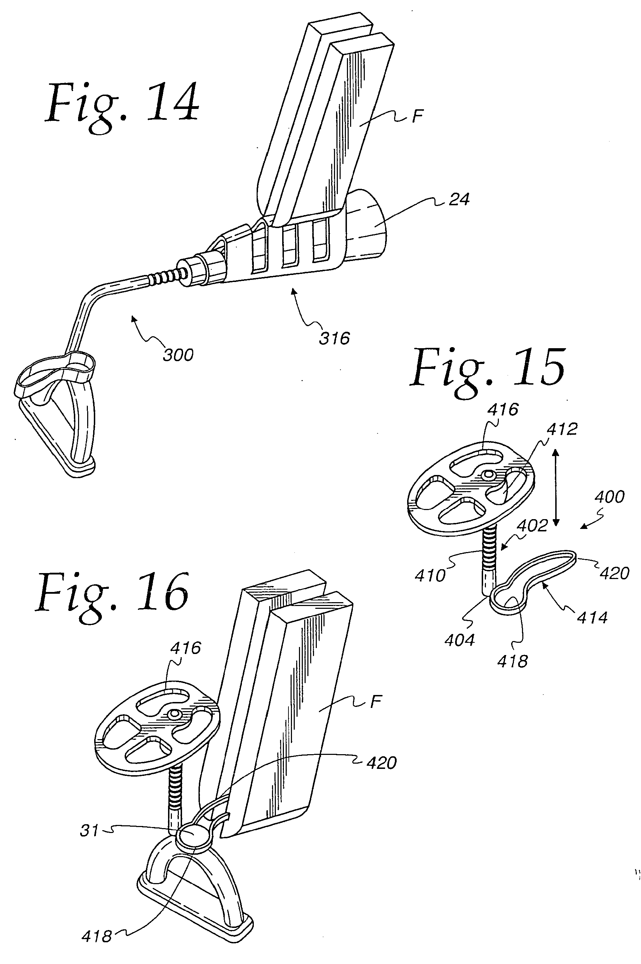

Referring to Fig. 13, a prosthesis 300 in accordance with yet another embodiment of the invention for necrosis applications is illustrated. The prosthesis 300 includes a shaft 302 similar to the shaft 202, discussed above, having a loop 314 at a first end 304. A silicon sleeve 312 is adjustably received on the shaft 302, as above. A hook in the form of a crimpable cage 316 is secured to the sleeve 312. The cage 316 differs from the cage 216 of Fig. 11 in that it is of one piece construction including a lower semi-cylindrical portion of gradually increasing diameter connected to crimp assist band strips 320, 322, 324 and 326 of successively larger size for capturing the incus 24, as shown in Fig. 14. The cage 316 may optionally be open along an upper ridge 328 of the band strips. In the illustrated embodiment of the invention the band cage 316 uses closed loops. Forceps F are used to crimp the band strips 320, 322, 324 and 326 to close the cage 316 around the remnant incus 24 providing crimping action outside of the circular or cylindrical area receiving the incus 24 so that it does not apply pressure directly over the incus 24 but only over the crimp assist band strips 320, 322, 324 and 326.

The cage 316 may be formed of a malleable titanium or other material, as discussed above.

Referring to Fig. 15, an adjustable length ossicular prosthesis 400 for partial repair or replacement of the ossicular chain is illustrated. The prosthesis 400 includes an elongate shaft 402 having a first end 404 connected to a hook in the form of a loop 414. A corrugated portion 410 adjustably receives a silicon sleeve 412 secured to a platform or head 416. The head 416 is for contacting a tympanic membrane when implanted in a human ear. Particularly, the platform 416 and adjustable feature are generally similar to that described in the U.S. Patent No. 6,168,625, incorporated by reference herein. The prosthesis 400 differs from the prior prosthesis in the use of the loop 414 for attaching to the stapes head 31. The loop 414 includes a circular portion 418 and a crimp assist portion 420. As illustrated in Fig. 16, the forceps F crimp the crimp assist portion 420 with the stapes head 31 received in the circular portion 418.

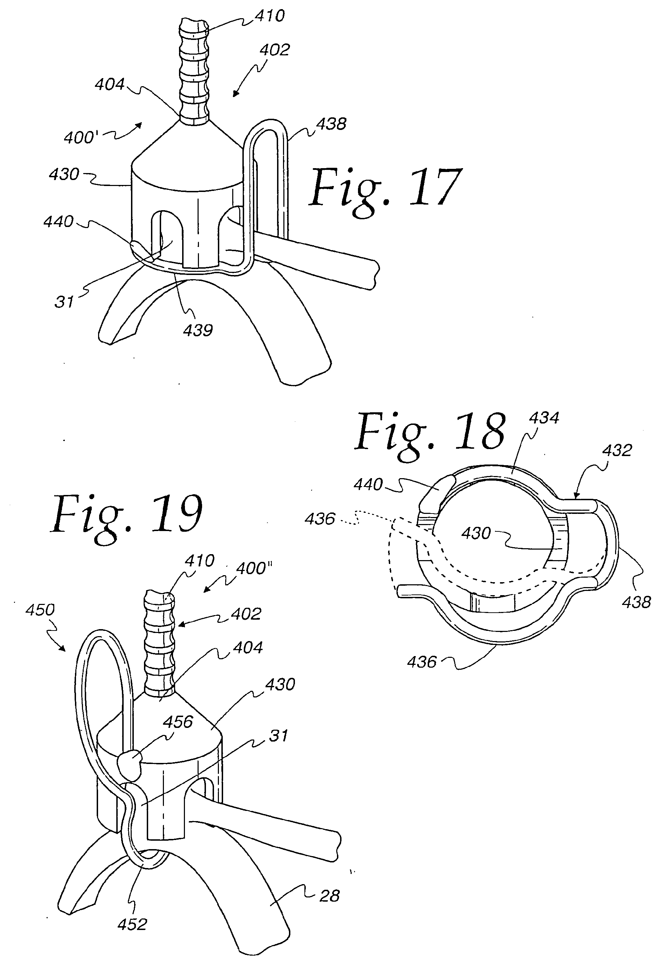

Figs. 17 and 18 illustrate an alternative prosthesis 400' for partial repair or replacement. The prosthesis 400' eliminates the loop 414 of Fig. 15 and replaces it with a cup 430 secured at the shaft first end 404. The cup 430 is receivable on the stapes head 31, as shown. A hook 432 is secured to the cup 430. The hook 432 includes opposite arcuate portions 434 and 436 connected by an out of plane crimp assist portion 438. The first arcuate portion 434 is secured to the cup 430 as by spot welding as at 440. When the crimp assist portion 438 is crimped, the second arcuate portion 436 closes around the stapes head 31, as shown in dashed line in Fig. 18 to secure the prosthesis.

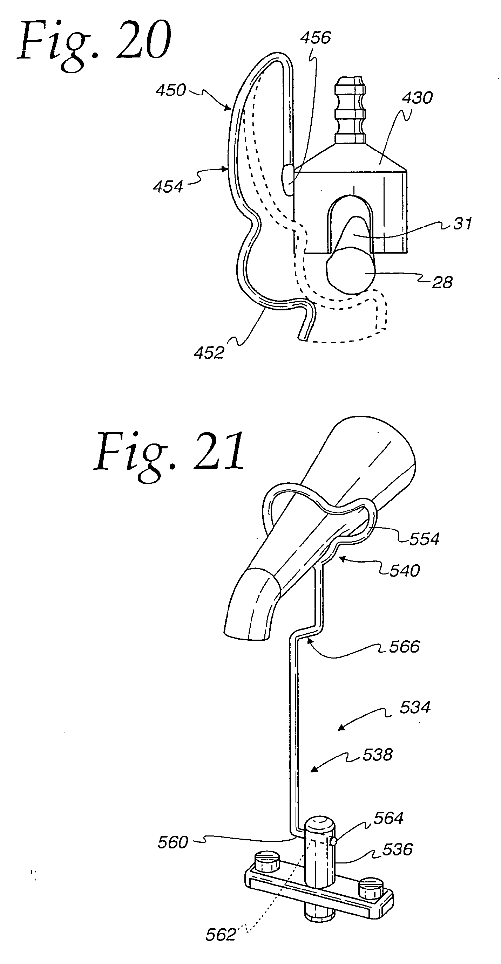

Figs 19 and 20 illustrate another alternative prosthesis 400" for partial repair or replacement. The prosthesis 400" eliminates the loop 414 of Fig. 15 and also replaces it with a cup 430 secured at the shaft first end 404. The cup 430 is receivable on the stapes head 31, as shown. A hook 450 is secured to the cup 430. The hook 450 includes an arcuate portions 452 connected by an out of plane crimp assist portion 454. The crimp assist portion 454 is secured to the cup 430 as by spot welding as at 456. When the crimp assist portion 454 is crimped, the arcuate portion 452 closes around the stapes arch 28, as shown in dashed line in Fig. 20, to secure the cup 430 on the head 31. In this embodiment, the cup itself forms part of the loop or hook.

Referring to Fig. 21, a prosthesis 534 includes a pedestal base or piston 536 and a shaft 538 pivotally connected to the piston 536. Particularly, the shaft 538 has a turned end 560 extending through a radially extending opening 562 in the piston 536 and spot welded as at 564 to retain the piston 536 on the shaft 538. The shaft 538 and the piston 536 are adapted to replace the stapes, as above. The shaft 538 is double reverse bent as at 566 to provide an offset compared to the embodiment of Fig. 4. A hook 540 is secured to the shaft 538 opposite the piston 536. The hook 540 comprises a crimp assist portion 554, similar to the crimp assist portion 54, discussed above.

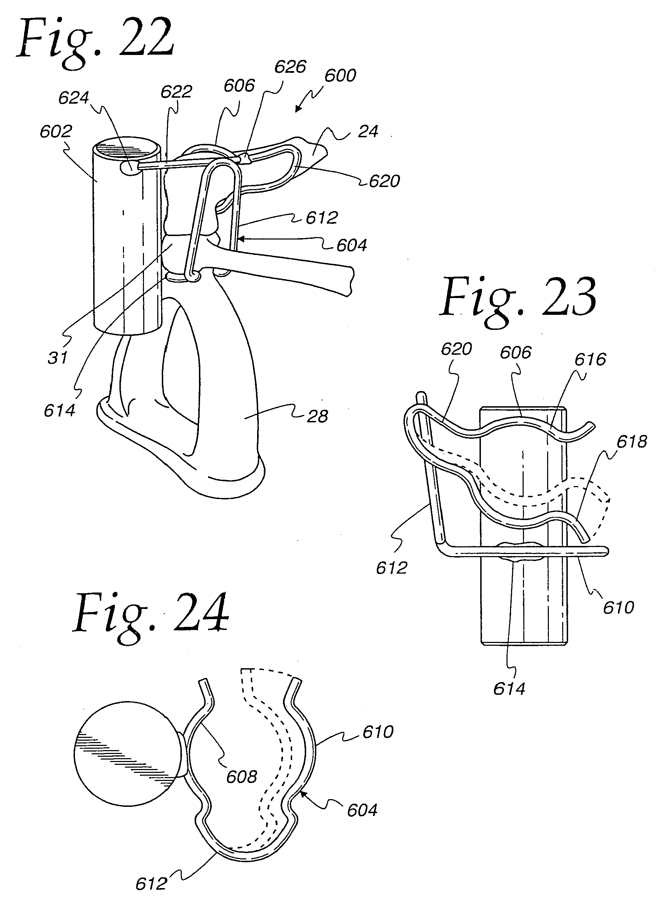

Figs 22, 23 and 24 illustrate a prosthesis 600 adapted to connect a titanium can 602 containing a magnet to the incus 24 or stapes head 31. The prosthesis 600 is not necessarily used for replacement, but can be used for hearing enhancement. The can 602 and magnet act as a transducer for a hearing enhancement mechanism. A piezoelectric device could also be used.

The prosthesis 600 includes a first hook 604 and a second hook 606. The first hook 604 includes opposite arcuate portions 608 and 610 connected by an out of plane crimp assist portion 612. The first arcuate portion 608 is secured to the can 602 as by spot welding as at 614. When the crimp assist portion 612 is crimped, the second arcuate portion 610 closes around the stapes head 31, as shown in dashed line in Fig. 24 to secure the prosthesis. The second hook 606 includes opposite arcuate portions 616 and 618 connected by a crimp assist portion 620. A brace 622 is spot welded to the can 602 as at 624 and to the second hook crimp assist portion 620 as at 626. When the crimp assist portion 620 is crimped, the second arcuate portion 618 closes around the incus 24, as shown in dashed line in Fig. 23 to secure the prosthesis.

Thus, the crimp assist prosthesis provides a means to crimp a wire of a middle ear prosthesis to the remnant bone of a middle ear. Providing the extended hook or loop in the form of the crimp assist portion, which the surgeon may crimp instead of crimping the wire directly to the remnant bone, simplifies the crimping procedure. This increases safety from a wire being crimped too tightly or too loosely to the remnant bone and is made of a desirable material such as titanium.

## Claims

1. A middle ear prosthesis comprising:
a member adapted to supplement an ossicular bone; and
an anatomically conforming structure secured to the member comprising an upper arm and a lower arm, each having near and distal ends, and a crimp assist portion connected to the near ends of the upper and lower arms so that the upper and lower arms generally define portions of a circle, oval, or the like, for partially surrounding a bone of the middle ear with the crimp assist portion offset therefrom, and the crimp assist portion can be crimped to close the structure around the bone of the middle ear without crimping within the circle, oval or the like.

2. A middle ear prosthesis according to claim 1, wherein the structure comprises a titanium wire.

3. A middle ear prosthesis according to claim 1 or claim 2, wherein the crimp assist portion is made from a relatively soft unalloyed titanium.

4. A middle ear prosthesis according to any preceding claim, wherein the member comprises a piston adapted to extend through an oval window when implanted in an ear and a shaft extending from the piston.

5. A middle ear prosthesis according to any preceding claim, wherein the crimp assist portion is angled at about 45° relative to an axis of the shaft.

6. A middle ear prosthesis according to claim 4 or claim 5, wherein the piston includes a depth indicator.

7. A middle ear prosthesis according to any preceding claim, wherein the distal ends of the arms are flared away from one another to define an enlarged opening.

8. A middle ear prosthesis according to any of claims 1 to 6, wherein the distal ends of the arms are joined to one another to define a closed loop.

9. A middle ear prosthesis according to claim 8, wherein the structure comprises a first loop and the member comprises a shaft having the first loop at a first end and a second loop, similar to the first loop, at a second end.

10. A middle ear prosthesis according to claim 9, wherein the second loop is adjustably positionable on the bent shaft at the second end.

11. A middle ear prosthesis according to any preceding claim, wherein the member comprises a crimpable cage.

12. A middle ear prosthesis according to any preceding claim, wherein the member comprises a shaft having a sleeve adjustably received on the shaft and a head secured to the sleeve.

13. A middle ear prosthesis comprising:
a member adapted to supplement an ossicular bone; and
an anatomically conforming structure secured to the member comprising an upper arcuate portion and a lower arcuate portion, each having near and distal ends, and a crimp assist portion connected to the near ends of the upper and lower arcuate portions so that the upper and lower arcuate portions generally define portions of a circle for partially surrounding a bone of the middle ear and the crimp assist portion extending outside of the circle, the circle in a pre-implant state having a diameter greater than diameter of a portion of the remnant bone, so that the crimp assist portion can be crimped to close the structure around the bone without crimping within the circle and after crimping the circle has a diameter to be retained on the bone.

14. A middle ear prosthesis according to claim 13, wherein the structure comprises a titanium wire.

15. A middle ear prosthesis according to claim 13 or claim 14, wherein the crimp assist portion is made from a relatively soft unalloyed titanium.

16. A middle ear prosthesis according to any of claims 13 to 15, wherein the member comprises a piston adapted to extend through an oval window when implanted in an ear and a shaft extending from the piston.

17. A middle ear prosthesis according to any of claims 13 to 16, wherein the crimp assist portion is angled at about 45° relative to an axis of the shaft.

18. A middle ear prosthesis according to claim 16 or claim 17, wherein the piston includes a depth indicator.

19. A middle ear prosthesis according to any of claims 13 to 18, wherein the distal ends of the arcuate portions are flared away from one another to define an enlarged opening.

20. A middle ear prosthesis according to any of claims 13 to 18, wherein the distal ends of the arcuate portions are joined to one another to define a closed loop.

21. A middle ear prosthesis according to claim 20, wherein the structure comprises a first loop and the member comprises a shaft having the first loop at a first end and a second loop, similar to the first loop, at a second end.

22. A middle ear prosthesis according to claim 21, wherein the second loop is adjustably positionable on the bent shaft at the second end.

23. A middle ear prosthesis according to claim 13, wherein the structure comprises a crimpable cage.

24. A middle ear prosthesis according to any of claims 13 to 23, wherein the member comprises a shaft having a sleeve adjustably received on the shaft and a head secured to the sleeve.

25. A middle ear prosthesis according to any of claims 13 to 23, wherein the member comprises a shaft having a cup receiving a head of a stapes and the structure is secured to the cup.

26. A middle ear prosthesis according to claim 25, wherein a portion of the cup defines the upper arcuate portion of the structure.

27. A middle ear prosthesis according to any of claims 13 to 15, wherein the member comprises a piston adapted to extend through an oval window when implanted in an ear and a shaft having first and second ends, the first end being secured to the structure and the second end being pivotally connected to the piston.

28. A middle ear prosthesis according to any of claims 13 to 15, wherein the member comprises a piston adapted to extend through an oval window when implanted in an ear and a shaft extending from the piston, the shaft being bent to provide an offset.

29. A middle ear prosthesis according to claim 13, wherein the crimp assist portion is in a different plane than the arcuate portions.

30. A middle ear prosthesis according to any of claims 13 to 29, wherein the member comprises a transducer.

31. A method of implanting a middle ear prosthesis comprising:
removing a portion of an ossicular chain;
providing a middle ear prosthesis comprising a member adapted to replace an ossicular bone, and an anatomically conforming structure secured to the member comprising an upper arcuate portion and a lower arcuate portion, each having near and distal ends, and a crimp assist portion connected to the near ends of the upper and lower arcuate portions so that the upper and lower arcuate portions generally define portions of a circle for partially surrounding an incus and the crimp assist portion extending outside of the circle, the crimp assist portion being of crimpable metal capable of retaining different shapes; and
implanting the prosthesis in the ear by placing the structure over a remnant bone and crimping the crimp assist portion to close the structure around the remnant bone without crimping within the circle.

32. A method according to claim 31, wherein the crimp assist portion is made from a relatively soft unalloyed titanium.

33. A method according to claim 31 or claim 32, wherein the crimp assist portion is angled at about 45° relative to an axis of the member.

34. A method according to any of claims 31 to 33, wherein the circle in a pre-implant state has a diameter greater than diameter of a portion of the remnant bone and after crimping the circle has a diameter to be retained on the remnant bone.

35. A method according to any of claims 31 to 34, wherein the crimp assist portion is generally U-shaped.
